# EUROPEAN PATENT APPLICATION

(11) **EP 1 520 538 A1**
(43) Date of publication of application: **06.04.2005**
(21) Application number: 04256022.7
(22) Date of filing: 30.09.2004
(51) Int. Cl.: A61B 17/34, A61B 19/02

(54) **Trocar assembly tip protector**

(30) Priority: 30.09.2003 US 506725 P; 17.09.2004 US 943219
(71) Applicant: ETHICON ENDO-SURGERY, INC., Cincinnati, Ohio 45242 (US)
(72) Inventor: Thompson, Brian J., Cincinnati Ohio 45226 (US); Gilker, Thomas A., Cincinnati Ohio 45211 (US); Jensen, Kurt, Lebanon Ohio 45036 (US); Albrecht, Thomas E., Cincinnati Ohio 45208 (US); Brewer, Jeffrey A., Hamilton Ohio 45011 (US); Barrow, Andrew E., Cincinnati Ohio 45208 (US)
(74) Representative: Tunstall, Christopher Stephen

(57) **Abstract**

A packaging device for a trocar assembly includes a housing member having a first retaining member adapted for selective retention of a trocar assembly component relative to the housing member along a component longitudinal axis. The housing member further includes a shield member remote from the first retaining member. The shield member lies in a shield longitudinal axis which is substantially perpendicular to the component longitudinal axis. The housing member is substantially open adjacent the shield member along the component longitudinal axis.

## Description

### CROSS REFERENCE TO RELATED APPLICATION

This application is based upon U.S. Provisional Patent Application No.60/506,725, filed September 30, 2003, entitled ''TROCAR ASSEMBLY TIP PROTECTOR", which is currently pending.

### BACKGROUND OF THE INVENTION

### 1. Field of the Invention

The invention relates to packaging devices for trocar assemblies. More particularly, the invention relates to a packaging device for trocar assemblies facilitating support of both a trocar sleeve and a trocar obturator while also providing for protection from the tips of the trocar sleeve and obturator.

### 2. Description of the Prior Art

A trocar assembly is a surgical instrument that is used to gain access to a body cavity. A trocar assembly generally comprises two major components, a trocar sleeve, composed of a trocar housing and a trocar cannula, and a trocar obturator. The trocar sleeve, having the trocar obturator inserted therethrough, is directed through the skin to access a body cavity. Once the body cavity is accessed, laparoscopic or arthroscopic surgery and endoscopic procedures may be performed. In order to penetrate the skin, the distal end of the trocar sleeve is placed against the skin that has been previously cut with a scalpel. The trocar obturator is then used to penetrate the skin and access the body cavity. By applying pressure against the proximal end of the trocar obturator, the sharp point of the trocar obturator is forced through the skin until it enters the body cavity. The trocar sleeve is inserted through the perforation made by the obturator and the obturator is withdrawn, leaving the trocar sleeve as an access way to the body cavity.

The proximal end portion of the trocar sleeve is typically adjoined by a housing that defines a chamber having an open distal end portion that communicates with the interior lumen defined by the trocar sleeve. An obturator, or other elongated surgical instruments, axially extend into and are withdrawn from the trocar sleeve through the proximal end portion of the chamber. Trocar obturators are typically designed with a sharp tip that is used to puncture the abdominal wall. Trocar packaging designs much ensure that the sharp tip of the trocar obturator does not puncture the packaging material that forms the sterile barrier. If the material used for the sterile barrier is not strong enough to prevent puncture on its own, an ancillary form of protection is required.

Typically, a tip protector is used for trocar assemblies, especially trocar obturators. Current tip protectors generally consist of a cap that completely encapsulates the sharp tip of the trocar obturator. Such caps completely obscure the view of the tip of the trocar obturator as it sits in the unopened package. It is, however, important for those using these instruments to be able to visibly identify the tip style prior to use. This is especially important when a variety of different obturator tip styles are available.

In addition to the tip problem discussed above, problems exist when providing packaging for surgical trocar assemblies. Some trocar assembly designs require that the trocar assembly be packaged with the obturator not inserted into the trocar sleeve. If this is the case, a two-piece device assembly must be packaged.

Surveys of operating room nurses responsible for removing trocar assemblies from their packages reveal that nurses prefer to dispense a single assembly from the trocar assembly package rather than a two-piece assembly. Reasons for preferring a one-piece assembly include reducing the opportunity for components to roll off from the sterile field as well as the desire to keep the obturator and trocar sleeve matched up together when dispensing or unpacking numerous different trocar types.

As a result, it is readily apparent that a trocar assembly packaging device is needed which overcomes the shortcomings of prior trocar assembly packaging devices. The present invention provides such a trocar assembly packaging device.

### SUMMARY OF THE INVENTION

It is, therefore, an object of the present invention to provide a packaging device for a trocar assembly. The packaging device includes a housing member having a first retaining member adapted for selective retention of a trocar assembly component relative to the housing member along a component longitudinal axis. The housing member further includes a shield member remote from the first retaining member. The shield member lies in a shield longitudinal axis which is substantially perpendicular to the component longitudinal axis. The housing member is substantially open adjacent the shield member along the component longitudinal axis.

It is also an object of the present invention to provide a trocar assembly including a packaging device. The trocar assembly includes a trocar sleeve having a trocar sleeve tip, a trocar obturator having a trocar obturator tip and a housing member. The housing member includes a first retaining member adapted for selective retention of the trocar sleeve along a first longitudinal axis and a second retaining member adapted for selective retention of the trocar obturator along a second longitudinal axis. The first and second longitudinal axes are substantially parallel. The housing member further includes a shield member remote from the first and second retaining members. The shield member lies in a shield longitudinal axis which is substantially perpendicular to the first and second longitudinal axes. The trocar sleeve tip and the trocar obturator tip are respectively positioned adjacent the shield member.

Other objects and advantages of the present invention will become apparent from the following detailed description when viewed in conjunction with the accompanying drawings, which set forth certain embodiments of the invention.

### BRIEF DESCRIPTION OF THE DRAWINGS

Figure 1 is a perspective view of a packaging device in accordance with the present invention.
Figure 2 is an alternate embodiment of a packaging device in accordance with the present invention.
Figure 3 is still a further embodiment of a packaging device in accordance with the present invention.
Figure 4 is a perspective view of the packaging device shown in Figure 2 with the trocar sleeve and trocar obturator attached.

### DESCRIPTION OF THE PREFERRED EMBODIMENTS

The detailed embodiments of the present invention are disclosed herein. It should be understood, however, that the disclosed embodiments are merely exemplary of the invention, which may be embodied in various forms. Therefore, the details disclosed herein are not to be interpreted as limiting, but merely as the basis for the claims and as a basis for teaching one skilled in the art how to make and/or use the invention.

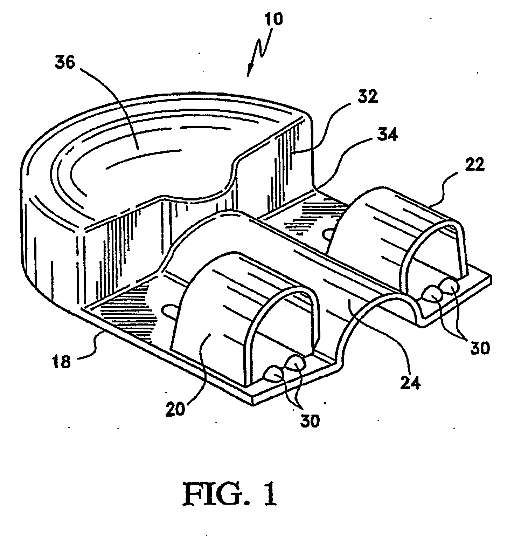

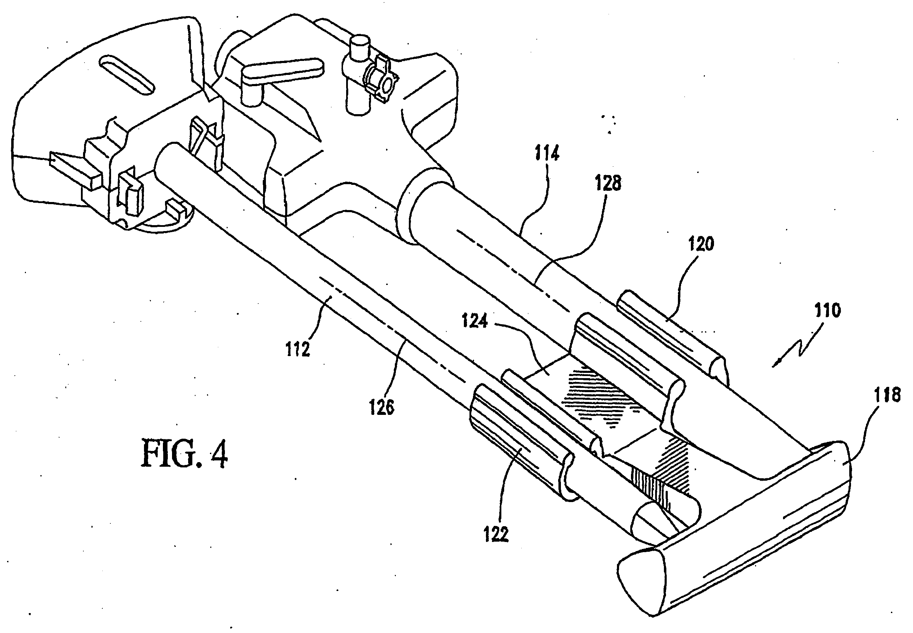

With reference to Figure 1, a packaging device 10 for trocar assembly components, for example, a trocar sleeve 12 and a trocar obturator 14 of a trocar assembly 16 (see Figure 4), is disclosed. The packaging device 10 includes a molded plastic housing member 18 that serves as a tip protector for trocar assemblies. The packaging device 10 further provides for supporting the trocar obturator 14 adjacent the trocar sleeve 12 in a convenient manner. While the present packaging device is disclosed in accordance with a preferred embodiment as being constructed from molded plastic, the packaging device may be manufactured from a variety of materials without departing from the spirit of the present invention.

The packaging device 10 includes a unitary housing member 18 with adjacent retaining members, for example, first and second sleeves 20, 22, mounted upon a base member, or bridge 24. The first and second sleeves 20, 22 are joined to each other via the base member 24. Briefly, the shafts of the trocar obturator 14 and trocar sleeve 12 are held parallel to each other when they are placed into the respective first and second sleeves 20, 22. That is, the first and second sleeves 20, 22 support the trocar obturator 14 and the trocar sleeve 12 along respective first and second longitudinal axes 26, 28 that are substantially parallel (see Figure 4).

In accordance with a preferred embodiment of the present invention, each of the first and second sleeves 20, 22 are shaped and dimensioned to frictionally hold the shafts of the trocar obturator 14 and/or the trocar sleeve 12. As such, the first and second sleeves 20, 22 are shaped and dimensioned to respectively frictionally engage the trocar obturator 14 and the trocar sleeve 12 as they are slid within the sleeves 20, 22. Frictional engagement of the trocar obturator 14 and the trocar sleeve 12 is enhanced by the provision of standoffs 30 along the base member 24. In addition to assisting in the frictional engagement of the trocar obturator 14 and trocar sleeve 12, the standoffs 30 respectively associate with each of the first and second sleeves 20, 22 create air space around the components, which is necessary for gas sterilization.

The tips of both the trocar sleeve 12 and the trocar obturator 14 are protected by a shield member 32 positioned forward of the first and second sleeves 20, 22 and extending upwardly from the forward end 34 of the base member 24. In fact, the housing member 18 is substantially open between the shield member 32 and the first and second sleeves 20, 22 along the component longitudinal axes 26, 28. The shield member 32 is shaped and dimensioned to function as a stop for the tips of the trocar obturator 14 and trocar sleeve 12. The shield member 32 further protects the tips from contacting the next layer of packaging material that may form a sterile barrier for the trocar assembly 16.

The packaging device 10, and particularly the shield member 32, is formed such that the tips of the trocar obturator 14 and the trocar sleeve 12 are covered only at their ends by the shield member 32 and the backsides of the trocar obturator 14 and trocar sleeve 12 are slightly covered by the base member 24, the first sleeve 20, the second sleeve 22 and to shield member 32. In fact, the base member 24 under the first sleeve 20 and the second sleeve 22 is open to enhance viewing of the trocar sleeve 12 and the trocar obturator 14. In addition, the spacing provides an open area surrounding the trocar obturator 14 and trocar sleeve 12 for sterilization and other purposes. Therefore, the front surfaces of the tips of the trocar obturator 14 and trocar sleeve 12 are unobscured and can easily be identified. In addition, the packaging device 10 includes a handle member 36 extending from the shield member 32. The handle member 36 is generally sized to permit ready handling by those using the present packaging device 10.

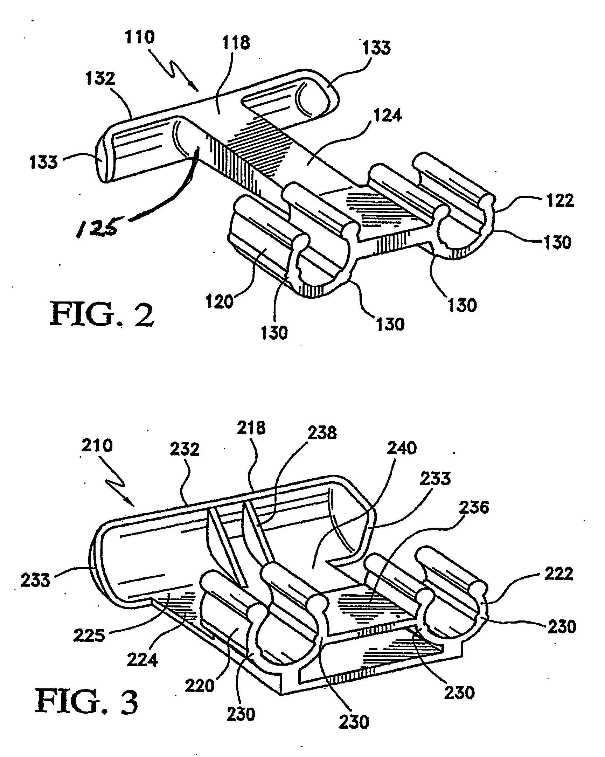

With reference to Figures 2 and 4, a further embodiment of a packaging device 110 in accordance with the present invention is disclosed. The packaging device 110 includes a molded plastic housing member 118 that serves as a tip protector for trocar assembly 16, in particular, the trocar obturator 14 and trocar sleeve 12 of a trocar assembly 16. The packaging device 110 further provides for supporting the trocar obturator 14 adjacent the trocar sleeve 12 in a convenient manner. While the present packaging device 110 is disclosed in accordance with a preferred embodiment as being constructed from molded plastic, the packaging device 110 may be manufactured from a variety of materials without departing from the spirit of the present invention.

The packaging device 110 includes a unitary housing member 118 with adjacent retaining members, for example, first and second side-loaded clips 120,122. The first and second side-loaded clips 120, 122 are joined to each other via a bridge 124 of plastic material. Briefly, the shafts of the trocar obturator 14 and trocar sleeve 12 are held parallel to each other when they are placed into the respective first and second side-loaded clips 120, 112. That is, the first and second side-loaded clips 120, 122 support the trocar obturator 14 and the trocar sleeve 12 along respective first and second longitudinal axes 26, 28 that are substantially parallel.

In accordance with a preferred embodiment of the present invention, each of the first and second side-loaded clips 120, 122 are shaped and dimensioned to securely hold the shaft of a trocar obturator 14 and/or a trocar sleeve 12. As such, the first and second side-loaded clips 120, 122 are formed with a basic resilience allowing for selective insertion and retrieval of the trocar obturator 14 and trocar sleeve 12 to and from the packaging device 110. In addition, each of the first and second side-loaded clips 120, 122 includes standoffs 130 along the interior surface thereof. The standoffs 130 create air space around the components, which is necessary for gas sterilization.

The tips of both the trocar sleeve 12 and the trocar obturator 14 are protected by a shield member 132 perpendicularly oriented relative to the bridge 124 connecting the first and second side-loaded clips 120, 122. More specifically, the bridge 124 includes a connecting member 125 extending from the first and second side-loaded clips 120, 122. The connecting member 125 connects the shield member 132 to the first and second side-loaded clips 120, 122.

With this in mind, the connecting member 125 extends a sufficient distance to space the first and second side-loaded clips 120, 122 from the shield member 132. The ends of the bridge 124 forming the connecting member 125 turn outwardly, merging into the shield member 132 such that the longitudinal axis 26, 28 of a trocar sleeve or obturator 12, 14 mounted within the side-loaded clips 120, 122 extends perpendicular to the longitudinal axis of the shield member 132. The shield member 132 is shaped and dimensioned to function as a stop for the tips of the trocar obturator 14 and trocar sleeve 12. The shield member 132 further protects the tips from contacting the next layer of packaging material that may form a sterile barrier for a trocar assembly.

The packaging device 110, and particularly the shield member 132, are formed such that the tips of the trocar obturator 14 and the trocar sleeve 12 are covered only at their ends by the shield member 132 and the backsides of the trocar obturator 14 and trocar sleeve 12 are slightly cover by the bridge 124, the first side-loaded clip 120 and the second side-loaded clip 122. Therefore, the front surfaces of the tips are unobscured and can easily be identified. In addition, the shield member 132 includes wrap around side surfaces 133. The wrap around side surfaces 133 aid in containment of the tips of the trocar obturator 14 and the trocar sleeve 12 should there be any deflection of the tip protector under load.

An alternate embodiment of the packaging device 210 is disclosed with reference to Figure 3. As with the embodiment described above, the packaging device 210 includes a molded plastic housing member 218 that serves as a tip protector for the trocar obturator 14 and the trocar sleeve 12. The packaging device 210 also supports the trocar sleeve 12 and the trocar obturator 14 adjacent each other. The packaging device 210 further includes a unitary housing member 218 with adjacent retaining members, for example, first and second clips 220, 222. The first and second clips 220, 222 are joined to each other via a bridge 224 of plastic material and the shafts of the trocar obturator 14 and trocar sleeve 12 are held parallel to each other when they are placed into the respective first and second clips 220, 222.

However, and in contrast to the embodiment described above, the first and second clips 220, 222 are connected via a bridge 224 that extends between and beneath the first and second clips 220, 222. As such, the bridge 224 generally functions as a base upon which the first and second clips 220, 222 are mounted.

In addition, and as with the previously disclosed embodiment, each of the first and second clips 220, 222 includes standoffs 230 along the interior surface thereof. The standoffs 230 create air space around the components, which is necessary for gas sterilization.

The tips of both the trocar sleeve 12 and the trocar obturator 14 are protected by a shield member 232 perpendicularly oriented relative to the bridge connecting the first and second clips 220, 222. However, and in contrast to the prior embodiment, the shield member 232 extends upwardly from the surface of the forward end 225 of the bridge 234. The shield member 232 is shaped and dimensioned to function as a stop for the tips of the trocar obturator 14 and trocar sleeve 12. The shield member 232 further protects the tips from contacting the next layer of packaging material that may form a sterile barrier for the trocar assembly.

The shield member 232 includes wrap around side surfaces 233. The wrap around side surfaces 233 aid in containment of the device tips should there be any deflection of the tip protector under load. The rigidity of the present packaging device 210 is enhanced by providing an upper secondary bridge 236 between the clips 220, 222. This upper secondary bridge 236 increases the rigidity and fixation of the components in relation to each other. The packaging device 210 further includes struts 238 connecting the bottom surface 240 of the bridge 224 to the shield member 223. The struts 238 provide rigidity when the packaging device 210 is under load and during removal of the trocar components from the packaging device 210.

While the preferred embodiments have been shown and described, it will be understood that there is no intent to limit the invention by such disclosure, but rather, is intended to cover all modifications and alternate constructions falling within the spirit and scope of the invention as defined in the appended claims.

## Claims

1. A packaging device for a trocar assembly, comprising:
a housing member including a first retaining member adapted for selective retention of a trocar assembly component relative to the housing member along a component longitudinal axis;
the housing member further including a shield member remote from the first retaining member, the shield member lying in a shield longitudinal axis which is substantially perpendicular to the component longitudinal axis; and
wherein the housing member is substantially open adjacent the shield member along the component longitudinal axis.

2. The packaging device according to claim 1, further including a second retaining member adjacent the first retaining member for selective retention of a trocar assembly component.

3. The packaging device according to claim 2, wherein a bridge connects the first retaining member to the second retaining member.

4. The packaging device according to claim 3, wherein the bridge connects the first and second retaining members to the shield member.

5. The packaging device according to claim 4, wherein the bridge extends between the first retaining member and the second retaining member.

6. The packaging device according to claim 4, wherein the bridge is connected to an underside of the first retaining member and the bridge is connected to an underside of the second retaining member, defining a base for the first and second retaining members.

7. The packaging device according to claim 4, wherein the bridge connects to a center of the shield member.

8. The packaging device according to claim 4, wherein the bridge connects to a bottom edge of the shield member.

9. The packaging device according to claim 1, wherein the housing member is a unitary member.

10. The packaging device according to claim 1, wherein a handle extends from the shield member.
